# EUROPEAN PATENT SPECIFICATION

(11) **EP 3 220 972 B1**
(45) Date of publication and mention of the grant of the patent: **29.04.2020**
(21) Application number: 15860892.7
(22) Date of filing: 13.11.2015
(51) Int. Cl.: A61M 1/00, A61M 25/00, A61M 39/10, A61M 39/22, A61M 39/26, A61M 39/02, A61M 39/28, A61M 5/178, A61M 3/02

(54) **DEVICES AND METHODS FOR DRAINAGE, INFUSION, OR INSTILLATION OF FLUIDS**
VORRICHTUNGEN UND VERFAHREN ZUR DRAINAGE, INFUSION ODER INSTILLATION VON FLÜSSIGKEITEN
DISPOSITIFS ET PROCÉDÉS DE DRAINAGE, DE PERFUSION OU D'INSTILLATION DE FLUIDES

(30) Priority: 21.11.2014 US 201462083142 P
(43) Date of publication of application: 27.09.2017
(73) Proprietor: Merit Medical Systems, Inc., South Jordan UT 84095 (US)
(72) Inventor: DOLMATCH, Bart, Palo Alto, CA 94306 (US); BAGAOISAN, Celso, Union City, CA 94587 (US); PAI, Suresh Subraya, Los Altos, CA 94022 (US); KOMLOS, Fabio, Los Altos, CA 94024 (US)
(74) Representative: Ricker, Mathias
(86) International application number: PCT/US2015/060749
(87) International publication number: WO 2016/081323

(56) References cited:
- CN-U- 202 554 582
- CN-Y- 2 691 631
- JP-A- 2004 008 238
- US-A- 5 084 031
- US-A- 6 099 511
- US-A1- 2009 143 731
- US-A1- 2011 190 710
- US-A1- 2013 060 205
- US-A1- 2014 155 744
- US-B1- 6 547 753

## Description

### FIELD OF THE INVENTION:

The present invention relates to devices and systems for draining fluids from a patient's or other user's body, and, more particularly, relates to valve devices and to systems for draining fluids, e.g., via a drainage catheter or tubing, that include such valve devices.

### BACKGROUND:

There are many reasons that external drainage of normal and abnormal body fluids or infusion or instillation of fluids or medications into the body must be performed. Some of these conditions include the need for drainage of pus, stool, urine, bile, serous fluid, lymph, gastric or enteric contents, or blood; the need for instillation or infusion of fluids or medications into organs such as the stomach or intestines, renal collecting systems, biliary tree, abscess cavities, seromas, lymphoceles, hematomas, bladder, chest cavity, or peritoneal cavity. Often, surgical and image-guided procedures leave an indwelling tube (drain or drainage, infusion, or instillation catheter) that passes from the internal bodily structures or collections through the skin to an external drainage, infusion, or instillation system. The main components of a typical external drainage, infusion, or instillation system (beyond the drainage, infusion, or instillation catheter) generally comprises tubing, a reservoir for the collection of the bodily fluid, and often a valve that permits control and restricts or permits access to the drainage, infusion, or instillation system. The tubing may comprise any sort of flexible conduit with at least one lumen along its length through which the bodily fluid passes. The collection reservoir may comprise a simple bag (e.g., that allows drainage, infusion or instillation by passively or via the assistance of gravity) or a suction system whereby bodily fluid is withdrawn from the body via the assistance or urging of negative pressure or a vacuum force.

While a typical drainage, infusion, or instillation catheter is in place, free flow of bodily fluid through the system must be assured. As fluid from many types of internal body fluids and collections often contain debris, clot, mucous, coagulated proteins, stones, or other substances that can obstruct the tubing, it is not uncommon that said fluids sometimes inhibit or prevent unimpeded drainage, infusion, or instillation of bodily fluid. Free flow into the body can also be reduced or obstructed by viscosity or materials in the system. This can render the drainage, infusion, or instillation system less functional or sometimes useless, and therefore the underlying problem may not be treated as clinically indicated or desired by the physician. When this occurs, the dwell time or residence of such a drainage, infusion, or instillation catheter in the patient's body may be prolonged and patient safety and comfort will be compromised. To combat this issue and to maintain flow in the drainage, infusion, or instillation system, a valve is often placed in the line between the drainage, infusion, or instillation catheter and the reservoir or delivery system. This valve facilitates easy injection of sterile flush, fluids (e.g., saline), or medications to clear the line. The valve may also permit aspiration of debris or thick/viscous secretions, repeated irrigation or lavage of the internal fluid collection to clear the system of obstructing material, and instillation of fluids such as enteral feeding or enteral medications.

Ideally, the valve used in this system should be simply and easily operated by both patients and health care professionals. Often, patients care for their drainage, infusion, or instillation systems at home, and they must use the valve every day. In some cases, this must be done more than once each day by the patient. Typically, to operate the valve there is some mechanism that allows flow to be diverted to a side port to which a standard slip luer or luer lock syringe can be attached for aspiration of the drainage, infusion or instillation of fluid or instillation of sterile fluids, or medications.

A standard valve that is placed in a drainage or infusion/instillation system is typically a "three-way stopcock" well known in the art. A three-way stopcock is a flow diverting device with three separate ports oriented so that by turning or rotating an internal cam with discrete flow-through channels, fluid can drain from the drainage source to infusion or instillation catheter and finally to a reservoir; or that fluid or medications can be instilled into the system. By rotating the cam within the stopcock, flow can be diverted to a port that permits the attachment of a standard slip luer or luer lock syringe for aspiration or injection of fluid or medications into the system. Three-way stopcocks have an asymmetric three-pronged lever that is attached to the cam so that the operator of said stopcock could easily turn the cam. This lever is used to determine the direction of flow in whichever position the cam has been turned.

However, the direction of flow related to the position of the pronged lever on most three-way stopcocks is not intuitive to many patients or health care providers. It is very possible to leave the cam set to an incorrect direction of flow, to leave the cam turned so that there is no flow (for example, incorrectly positioned between ports), or to inadvertently leave the flow externally vented. Flow direction and diversion related to a three-way stopcock relies upon exact positioning of the channels within the cam so that they have an alignment with the channels that pass through the three ports of the stopcock. If the cam is turned out of position, the channels within the cam may not align with the ports, and flow will be halted from every direction. If the cam is turned so that its channels are misaligned (i.e., not coaxially aligned together), there will be a smaller lumen for flow of fluid (i.e., a constriction at the interface of the cam's channel and the port's lumen) impeding desirable flow though. Also, if there is misalignment (and therefore constriction) then a very small amount of debris can potentially cause complete obstruction to flow and a minor degree of misalignment can render the entire drainage, infusion, or instillation system useless.

Malpositioning of the three-way stopcock cam relative to its ports is frequently observed, even when the stopcock is used by trained health professional (e.g., nurses, physician's assistants, and physicians). In addition, since most use of the aforementioned three-way stopcocks is done by a patient at home, the potential opportunity for such malpositioning is significant since patients may be unaware or comprehend the internal structure of the stopcock's cam and ports and as such are unlikely to know that precise alignment of the cam's flow channels to the port lumens is of critical importance to the overall and desirable functioning of the stopcock. Since there may be few interval visits to a clinic or hospital for such patients, flow problems of the drainage, infusion, or instillation system due to stopcock misalignment issues outside of a health care facility may go on for protracted periods (sometimes days or even weeks) without recognition of the issue, subjecting such patients to increased morbidity, potential safety issues, and/or clinical sequelae and/or longer than necessary periods of treatment and follow-up.

Even when the cam is turned or rotated into its exact desired position following usage for aspiration or infusion, it is very possible that the attached external pronged lever can be displaced slightly (i.e., rotated out of position) by inadvertent force causing misalignment or obstruction of the flow channels. This can happen if the three-way stopcock is rubbed against something, knocked slightly, or lies between two frictional surfaces such that the cam is turned out of alignment, which is not uncommon in such systems since the patients go about their normal daily lives and routines (sleeping, dressing, etc.). Since the three-way stopcock is external to the patient and positioned between the patient's skin and clothing, it is easily understood and anticipated that the stopcock lever can likely be twisted or rotated simply by catching on the patient's clothing or being rubbed between the patient and a seat back or bed. Inadvertent mechanical malpositioning of a three-way stopcock is a serious deficiency of this type of valve since it can render the entire drainage, infusion or instillation system useless.

It is further notable that flow reduction is inherent in many three-way stopcocks even when the flow channels of the rotating cam are in perfect alignment with the port lumens. This is because the flow channels and lumens of most three-way stopcocks are significantly smaller than the smallest luminal diameter of all of the other tubular components used in a typical drainage, infusion, or instillation system. That is, the lumens of the drainage, infusion, or instillation catheter or tubing, as well as the access lumen or port of the reservoir can be larger than the channels and ports of the three-way stopcock. Therefore, the three-way stopcock, even when set optimally with the cam positioned appropriately, may still produce a relative constriction to flow due to this dimensional difference. The flow channels and smaller diameter lumens found in most stopcocks likely present bottlenecks or sites for entrapment of obstructing materials (as previously described) because of their relatively smaller diameter compared with the rest of the drainage, infusion, or instillation system. Thus, these flow channels and luminal spaces within a stopcock may be the site where material may be trapped or lodged and completely obstructs flow in the drainage, infusion, or instillation system.

Three-way stopcocks have protruding and angular components usually fabricated from injected molded hard plastic materials (e.g., ABS, polycarbonate, and the like) that, when pressed against the patient's skin or body, can indent, excoriate, abrade, and even ulcerate the skin. This can pose risk of inflammation and even infection including dermatitis, necrosis, cellulitis, and even subcutaneous and deep abscesses and infections. Beyond the medical risks, the pressure from the protruding components of a stopcock can also cause significant pain, can get caught in clothing and other external objects, and pose risk for dislodgement of the drainage, infusion, or instillation catheter from the patient. Discomfort from lying on such a stopcock can interfere with the patient's ability to sleep or obtain adequate rest further compromising the patient's health and well-being. Sometimes the catheter can even become partially dislodged resulting in an inappropriate position for the catheter in the body for optimal drainage, infusion or instillation.

Traditional three-way stopcocks have a number of other mechanical failure modes that pose risk to the patient, caregivers, family and friends, as well as potential to compromise the overall effectiveness of the drainage, infusion, or instillation system. The rotatable cam can be partially dislodged from its housing by external lateral force so that it unintentionally vents fluid externally while ostensibly being intact. In addition, the rotatable cam can potentially be entirely dislodged out of the stopcock housing and usually cannot be replaced, leaving ALL ports of the stopcock housing communicating with each other in the space where the cam once sat. Regardless of partial or complete cam dislodgment, when any degree of cam dislodgment happens, fluid can leak out of the drainage or infusion/instillation system from either the drainage, infusion, or instillation catheter or by reflux from the drainage, infusion, or instillation tubing. This fluid can leak onto the patient or into their environment and pose a risk of spread of contaminated fluids to the patient or people who may come in contact with the patient's pus, stool, urine or other contaminated fluids.

Beyond dislodgement of the rotatable cam, it is also possible that one or more of the ports of the stopcock can snap off when too high a force or leverage is applied. For example, this type of undesirable force can be produced in normal use when an attached syringe acts to snap the port off of the stopcock by inadvertent application of too high a torque and/or angular force. The drainage, infusion, or instillation catheter port or drainage, infusion, or instillation tubing port can also snap off if too high of an oblique force is manually applied from the tubing to the stopcock, again generating significant shearing force at the base of the port. Finally, if a vacuum drainage system is engaged and the stopcock's rotatable cam is unintentionally turned to divert flow from a port to the reservoir during active suction, the vacuum may be lost and again the system will be rendered ineffective.

US 2009/14373 A1 discloses a kit for charging an infusion pump having a reservoir for holding a local anesthetic. The kit includes a first stopcock having an input port, a first input/output port and a second output port and a manual control for selectively coupling either the first input/output port or the second output port to the input port. The kit further includes a second stopcock having an input port, first and second output ports and a manual control for selectively coupling either the first output port or the second output port to the input port. The second output port of the first stopcock is adapted for coupling to the input port of the second stopcock. The kit further includes a syringe adapted to be coupled to the first input/output port of the first stopcock and tubing for coupling the input port of the first stopcock to a source of the liquid and for coupling the output port of the second stopcock to an input port of the pump.

US 2013/060205 A1 discloses a valve that has a body with an inlet, an outlet, and a needleless access port coupled to the body. The access port has an interior space and is configured to be actuated by a needleless connector. The inlet is selectively coupled to the outlet through the interior space such that fluid entering the inlet entirely flows through the interior space to the outlet.

US Patent 6,099,511 discloses a manifold that has a manifold body defining a fluid flow pathway therethrough. The manifold body has a plurality of valves, at least one of which is a check valve. The manifold is coupled at one end to a syringe or other fluid delivery means and to an opposing end to a catheter or other fluid receiving means. By being positioned within the manifold body, the check valve is reinforced and stabilized within the manifold.

US 2011/190710 A1 relates to a therapeutic solution of an amount corresponding to a plurality of injections is injected with the distal end kept inserted in the pericardial cavity via an access port formed to penetrate under the xiphoid process. Provided is a therapeutic-solution injecting device including an injection needle for puncturing living tissue; a tube having the injection needle at a distal end thereof; a syringe connector provided at a proximal end of the tube and allowing connection and disconnection of a therapeutic-solution syringe containing therapeutic solution; and a three-way valve provided in the tube between the syringe connector and the injection needle and having an open end.

US 2014/155744 A1 discloses a power injection catheter assembly which includes a power injection catheter having an elongate tubular body. The elongate tubular body has a proximal end and a distal end and defines a power injection lumen A manifold is attached at the proximal end of the elongate tubular body and defines a first power injection channel extending from a first power injection port of the manifold to the power injection lumen, and a second power injection channel extending from a second power injection port of the manifold to the power injection lumen. A first power injection extension tube has a proximal end defining a first power injection hub and a distal end attached to the manifold at the first power injection port. A second power injection extension tube has a proximal end defining a second power injection hub and a distal end attached to the manifold at the second power injection port.

Thus, with these notable shortcomings, it is apparent there is a need for improved devices and methods for drainage, infusion, and instillation of fluids in a body.

### SUMMARY:

The present invention is directed to devices and systems for draining fluids from a patient's or other user's body, and, more particularly, is directed to valve devices and to systems and methods for draining fluids, e.g., via a drainage catheter or tubing, that include such valve devices.

Described herein are devices to connect the various components of a drainage or infusion/instillation system along with methods of use of the same. The devices generally comprise a housing, two or more ports joined with a hollow member or members resident within the housing, and at least one means of reversibly restricting or closing the hollow member.

In accordance with an exemplary embodiment, a valve device is provided for draining fluids from and/or infusing fluids into a patient's body that includes a housing comprising first, second, and third connectors, and a primary fluid path communicating between the first and second connectors and a secondary fluid path communicating from the primary fluid path to the third connector, wherein the third connector is configured to prevent fluid flow therethrough unless a mating connector is fully coupled thereto; a first valve member movable within the housing between a first position wherein the first valve member does not obstruct the primary fluid path and a second position wherein the primary fluid path is closed to prevent fluid flow through the first connector; and a second valve member movable axially within the valve body between a first position wherein the second valve member does not obstruct the primary fluid path and a second position wherein the primary fluid path is closed to prevent fluid flow through the second connector.

In accordance with another embodiment, a system is provided for draining fluids from and/or infusing fluids into a patient's body that includes a valve device comprising a housing comprising first, second, and third connectors, a primary fluid path communicating between the first and second connectors and a secondary fluid path communicating from the primary fluid path to the third connector, wherein the third connector is configured to prevent fluid flow therethrough unless a mating connector is fully coupled thereto, the valve device further comprising first and second valve members for selectively closing flow along the primary fluid path through the first and second connectors, respectively; a tubular member comprising a first end sized for implantation and/or insertion into a patient's body and a second end comprising a tubular member connector configured to couple to the first connector; and a container for storing fluid from the patient's body comprising tubing including a container connector configured to couple to the second connector, thereby providing a fluid path from the tubular member through the primary fluid path and tubing into the container.

In accordance with still another unclaimed embodiment, a method is provided for draining fluids from and/or infusing fluids into a patient's body that includes providing a valve device comprising first, second, and third connectors, a primary fluid path communicating between the first and second connectors and a secondary fluid path communicating from the primary fluid path to the third connector, wherein the third connector is configured to prevent fluid flow therethrough unless a mating connector is fully coupled thereto, the valve device further comprising first and second valve members for selectively closing flow along the primary fluid path through the first and second connectors, respectively; providing a tubular member comprising a first end implanted or inserted into a patient's body and a second end extending from the patient's body; coupling the tubular member second end to the first connector; coupling tubing from a container to the second connector, thereby providing a fluid path from the tubular member through the primary fluid path and tubing into the container; coupling a source of at least one of vacuum and inflation media to the third connector to open the secondary flow path between the primary fluid path and the source; and activating at least one of the first and second valve members to close at least a portion of the primary fluid path.

Exemplary embodiments provided herein may include, but are not limited to, one or more of the following features:
a. Devices that have a generally low profile and smooth contours/surfaces and interfaces that are specifically designed with the intent to mitigate the potential for pain and wound creation if the device is pressed against the skin, as well as to facilitate usage and overall wearability underneath clothing without being too cumbersome or noticeable.
b. Devices that provide a simple and easy logic interface as it relates to infusion, instillation, and/or drainage in a form factor that is easily understood and utilized by healthcare professionals, other caregivers, as well as patients.
c. Devices that include features that make them foolproof with the intent of preventing inadvertent malpositioning and/or accidental restriction of flow and/or accidental drainage of contaminated fluids onto the patient or into the environment about the patient.
d. Devices that include lumens and ports having dimensions that specifically optimize and maximize flow of bodily fluids and medications while minimizing the opportunity to become obstructed by debris.
e. Devices that have a general aesthetic appeal intended to reduce patient's embarrassment or anxiety related to such devices, facilitate usage and reduce the encumbrance to the patient's day-to-day life and routines.

In one embodiment, the device generally comprises at least one elongate member comprising at least one lumen extending through the length of the elongate member. The inner diameter of the at least one lumen of the elongate member may be sized to accommodate the exudate, body fluid, or other material that the device is intended to drain. The elongate member or members may have identical or dissimilar numbers of lumens, and the inner diameter of the lumens may be identical, dissimilar, or combinations thereof. Similarly, the outer diameters and lengths of the elongate member or members may be identical, dissimilar, or combinations thereof. Further, the wall and/or the outer diameter of the elongate member or members may be constant or variable in dimension.

The elongate member or members may further comprise a fixed or variable stiffness over the length of each elongate member, and a particular stiffness or variation in stiffness along the length of the elongate members may be identical or dissimilar, or a combination thereof. The elongate member or members are constructed in such a manner that a force applied to the external surface of the elongate member above a threshold value will collapse the at least one lumen and restrict the path between the two ends of the elongate member.

The elongate members may be fabricated from materials known in the art including, but not limited to one or more of, aliphatic polyamides, fluorinated ethylene propylene, nylon, perfluoroalkoxy (e.g., Teflon®), polyether block amide (Pebax®), polyetheretherketone (PEEK), polyethylene, polytetrafluoroethylene (PTFE), polypropylene, polyurethane, polyvinylchloride, natural rubber, nitrile rubber, silicone rubber, combinations and copolymers thereof, and the like. The composition of the elongate members may be identical, dissimilar, or combinations thereof.

The device may further comprise a connector that joins at least two elongate members, if desired. In one example of this configuration, the connector comprises a straight lumen extending through the length of the connector. The axis of this lumen, herein referred to as the primary lumen, may be considered the primary axis of the connector. The connector may further comprise a second straight lumen that intersects with the primary lumen. The angle between the primary lumen and the secondary lumen may range from one to one hundred eighty degrees (1-180°). As an example, a ninety degree (90°) angle between the primary and secondary lumens results in the internal space of the connector assuming a "T" shape. While this example has been presented with the assumption of straight primary and secondary lumens, it should be clear to one of skill in the art that either the primary and/or secondary lumens may have alternative geometries (e.g., curved, sinusoidal, etc.). In another example, the connector comprises three lumens that are joined at a center point in the shape of a "Y". The angles between the three lumens may be equal, not equal, or any permutation thereof (e.g., 120°/120°/120°, or 45°/45°/270°, or 60°/120°/180°, etc.).

While the examples described herein are provided under the assumption that the multiple lumens of the connector all lie on the same geometrical plane, permutations of the connector that comprise lumens in multiple different planes joining at a single point (e.g., three lumens that lie on the x, y, and z axes respectively and are joined at the origin) are contemplated as well. The internal diameter of the lumens of the connector may be sized to provide a smooth and continuous joint with the lumen of the elongate members. Alternatively, the lumens of the connector may be larger or smaller than the lumen of the elongate members. The connector may be fabricated from materials known to the art including, but not limited to, aliphatic polyamides, fluorinated ethylene propylene, nylon, perfluoroalkoxy (e.g. Teflon®), polyether block amide (Pebax®), polyetheretherketone (PEEK), polyethylene, polytetrafluoroethylene (PTFE), polypropylene, polyurethane, polyvinylchloride, natural rubber, nitrile rubber, silicone rubber, stainless steel, nickel, titanium, polycarbonate, acrylic, polyoxymethylene (Delrin®), combinations thereof, and the like.

In the housing of a connector comprising a "T" shaped internal lumen structure, one end of each of the two elongate members of the device are joined to each arm of the "T" using methods known to the art including, but not limited to bonding, welding, ultrasonic welding, over-molding, threading/tapping, crimping, friction fitting with hose barbs or the like and/or combinations thereof, and the like. Alternatively, the two or more elongate members may be configured without the need for the previously mentioned connector or connectors. In this example embodiment, the elongate members would be constructed or fabricated as a unitary object using methods known in the art including, but not limited to molding, casting, welding, ultrasonic welding, thermoforming, dipping or dip coating, three dimensional printing and the like.

The device further comprises two fittings that are joined to the free ends of the elongate member or members using methods known in the art including, but not limited to, bonding, welding, ultrasonic welding, over-molding, threading/tapping, crimping, combinations thereof, and the like. The fitting may be configured to reversibly connect to standard medical couplings including, but not limited to, male or female luer-locks (fixed or rotating), male or female luer-slips, quick-disconnect fittings, hose barbs, internally threaded fittings, externally threaded fittings, flexible tubing, and the like. The fittings may be fabricated from materials known to the art including, but not limited to, polycarbonate, polyethylene, polyolefin, polypropylene, polytetrafluoroethylene, polysulfone, polyvinylchloride, polyoxymethylene (Delrin®), brass, stainless steel, nylon, perfluoroalkoxy (e.g., Teflon®), natural rubber, nitrile rubber, silicone rubber, combinations thereof, and the like.

The fittings may further comprise a lumen that extends the length of the fitting. In one example, the linear assembly of a first fitting, a first elongate member, the connector (if any), a second elongate member (if any), and a second fitting provides a continuous conduit for the flow of materials from the first fitting to the second fitting (with a secondary path for flow available through the remaining lumen of the connector (if any) to and through the second elongate member lumen (if any)).

Optionally, the device may further comprise a luer-activated (needleless) check valve. In an exemplary embodiment, the valve may be connected to one of the lumens of one of the elongate members. Alternatively, the outlet of the luer-activated valve may be directly joined to the remaining lumen of the connector or joined to the remaining lumen of the connector via an intermediary conduit. In the housing that the luer-activated valve is directly joined to the connector, the assembly of the connector and luer-activated valve may be a single component. The luer-activated valve functions such that flow is only possible through the valve to or from the elongate members when a male luer-style connector (e.g., luer-lock, luer-slip, etc.) is inserted into the inlet of the valve.

The device further comprises a housing that partially or fully encloses or encases the assembly of the fitting(s), elongate member(s), connector(s), and/or luer-activated valve(s), as previously described. The housing may be fabricated from materials known in the art including, but not limited to, polycarbonate, polyethylene, polyolefin, polypropylene, polytetrafluoroethylene, polysulfone, polyvinylchloride, polyoxymethylene (Delrin®), brass, stainless steel, nylon, perfluoroalkoxy (e.g. Teflon®), natural rubber, nitrile rubber, silicone rubber, combinations thereof, and the like. For example, a portion of a polycarbonate housing that is intended as a gripping surface may further comprise a silicone rubber pad or cladding to increase the coefficient of friction of that particular segment of the housing and provide a more stable handhold to the patient. The housing may be comprised of multiple pieces or components that are affixed to each other and/or the assembly.

In another example, the housing may comprise two symmetric shells that partially enclose the assembly and leave the inlet of the luer-activated valve and the free ends of the fittings exposed to facilitate connection of the luer-activated valve and/or fittings to other components of the drainage, infusion or instillation system. The shells of such a housing may be joined to each other using methods known to the art including but not limited to bonding, welding, ultrasonic welding, threading/tapping, friction fits, interference fits, snaps, combinations thereof, and the like. Alternatively, the housing may be asymmetric in shape.

The housing may be relatively narrow with respect to the length of the housing, providing a slim profile that does not catch on clothing or other items while the patient is active and is comfortable (i.e., doesn't present sharp or high projections) that would produce discomfort if laid upon when a user of said apparatus is sleeping. For example, the depth of the housing may be less than or equal to one third of the length of the housing. Additionally, the external surface of the housing may comprise features such as rounded or tapered edges, curved, sloped, or contoured surfaces, textured segments wherein the surface of the shell is rougher or smoother than surrounding areas, patterns of or combinations thereof, and the like to further improve patient comfort and ease of use.

Optionally, the device may additionally comprise one or more features that use visual or tactile cues to communicate information to the patient. Such communication features may include those known in the art including, but not limited to, inscription, decals, stickers, labels, printing, pad printing, use of varied colors (e.g., injection molds parts of differing colors), combinations thereof, and the like. For example, the housing may be fabricated using a material having a white color. The fitting that is intended to connect with the drainage or infusion/instillation catheter may be fabricated using a blue material, the fitting that is intended to connect with the vacuum source may be fabricated using a yellow material, and the exposed section of the luer-activated valve may be fabricated using a red material.

In addition or alternatively, arrows or other symbols may be inscribed or pad printed on the housing or onto the components of the system to instruct the patient on how to connect the device to the other components of the drainage, infusion or instillation system, how to restrict lumens, how to drain the device, etc. These examples are nonlimiting in nature, and it should be understood that a range of communication means have been contemplated.

Furthermore, optionally, the housing may additionally comprise openings, gaps, or spaces in the body of the housing. In one example, the openings may be configured to house valves that enable the patient to selectively compress one of the elongate members. The openings may be shaped to enable a variety of valves to be mounted on, slidably disposed within, or otherwise interact with invention. For example, a housing made of two shells (e.g., a front shell and a back shell) may further comprise two circular openings in the front shell. The openings may extend or project inwards of the shell thickness, forming a cylinder with the openings aligned and oriented perpendicularly over each of the elongate members.

Alternatively, an opening may be configured to allow a lanyard, tie, cord, cable, rope, string, or other means of securing the device to an external object (e.g., the patient) to be passed through the device. For example, the housing may comprise a loop that projects away from the body of the device, wherein the loop is sized to allow passage of a cord through the loop. In another example, the housing may comprise a tubular opening with proximal and distal ends. The proximal and distal ends may be located on the same or disparate faces of the housing to form an enclosed conduit sized to accept a means of securing the device to an external object.

In some cases, an opening may be positioned such that visual inspection of an internal feature of the device is possible. For example, an opening may be aligned with the length of one of the elongate members to enable the patient to confirm flow of exudate or other material through the elongate member. This opening may further be covered by a translucent or transparent material to enable view of the elongate member while preventing physical access to the interior of the device (e.g., to act as a sight glass). It should be clear to one skilled in the art that a feature such as a translucent or transparent window may be expanded to locations beyond those explicitly stated, and that alternative uses and positions of this feature have been duly contemplated.

Optionally, the housing may further comprise features that enable the housing to be carried by, on, or mounted to the patient. In one example, at least one face of the housing may comprise a loop or set of loops that are sized to enable the device to be carried on a belt, band, webbing, suspender, or other like object. In another example, at least one face of the housing may comprise a "U" shaped feature that extends away from the housing to enable the device to be hung on a belt, waistband, or like object. In yet another example, the housing may comprise a segment of adhesive that allows the device to be directly mounted onto the patients skin.

Alternatively, a holster or pocket may be fabricated from materials that comprise any or all of the following characteristics: lightweight, breathable, water resistant, elastic, waterproof, durable. The holster or pocket may further comprise one or more features to secure the device within the holster or pocket while providing access to the complementary parts of the drainage or infusion/instillation system (e.g., infusion lines, vacuum lines, drainage or infusion/instillation catheter lines, etc.). The device may be secured by a flap that encloses the device and is reversibly fixed in place using means known in the art including, but not limited to, ties, hook-and-loop fasteners (e.g., Velcro®), buttons, snaps, grommets, zippers, clips, buckles, combinations thereof, and the like. The holster or pocket may have opening, holes, or other passageways that allow the device to reside inside the holster or pocket while connected to the complementary parts of the drainage or infusion/instillation system.

The holster or pocket may further comprise one or more features to attach the holster directly to the patient, to the clothes of the patient, or to other items. For example, a lightweight, elastic material (e.g., spandex) may be fabricated into a pocket that securely holds the device of the invention and comprises multiple openings for the complementary parts of the drainage or infusion/instillation system. The multiple openings may be located at positions that generally correspond to the location of the fittings and luer-activated valve inlet of the device. The holster pocket may also have a double-sided adhesive suitable for adhering the pocket to the skin of a patient affixed to one or more sides of the pocket, enabling the patient to wear the device underneath clothing.

Alternatively, the holster or pocket may comprise at least one grommet and at least one lanyard (wherein the lanyard is passed through the grommet) to allow the pocket to be worn on the arm, about the neck, or the like. In another example, the holster may additionally comprise at least one length of webbing that is reversibly or permanently fixed to the holster, wherein the ends of the webbing comprise complementary halves of a hook-and-loop fastener. The at least one length of webbing may be passed around the arm, leg, waist, or the like of the patient and reversibly fixed in position by mating the two halves of the hook-and-loop fastener. In yet another example, the webbing of the prior example may be elastic.

Optionally, the device additionally may include one or more valves that interact with and selectively close the elongate members. In one example, a valve may be located external to the elongate member and may comprise a disk with a cylindrical pillar of a diameter less than that of the disk perpendicular to, concentrically oriented with, and/or descending from the surface of one side of the disk. The edge at the end of the pillar may be beveled, chamfered, rounded, or otherwise modified to reduce the sharpness of the edge. For example, the end of the pillar may possess a hemispherical surface. The valve of this example may be slidably disposed within a cylindrical opening in the front of the housing, wherein the opening is oriented perpendicular to and aligned within the centerline of one of the elongate members. Each cylindrical opening may comprise a rim located about the external surface of the shell and oriented concentric to the cylindrical openings. The rim may be sized to a diameter smaller than the cylindrical opening having a depth that forms a top and bottom side and a thickness projecting outwards such that the outer perimeter of the rim lies coincident to the surface of the cylindrical opening. The rim may interfere with the disk of the valve and prevent the valve from sliding out of the cylindrical opening of the shell. Alternatively, the top side of the rim may be elevated above the outer surface of the shell to recess the disk of the valve.

In a passive state, the end of the pillar (i.e., the point of tangency to the elongate member) rests on the outer surface of the elongate member and the top surface of the disk is in contact with the base or bottom of the rim. As the patient applies pressure to the disk (i.e., pushes the valve "button"), the valve advances towards the elongate member, compressing the walls of the elongate member, restricting and eventually closing the lumen of the elongate member. When the patient stops applying pressure to the disk, the elongate member resiliently and/or automatically recoils and returns to its original shape, re-opening the lumen of the elongate member and pushing the valve towards its original position until the disk contacts the bottom of the rim. This configuration of the valve apparatus prevents the opportunity to inadvertently leave the lumen of the elongate member in the closed or restricted state.

This is in stark contrast to the traditional three-way stopcocks wherein the valve maybe malpositioned or inadvertently left in a closed or restricted state. In this embodiment, the valve is positioned external to the flow path and thereby does not impede the lumen of the elongate member when the valve is in the open or non-restricting state. Here again, this configuration is in stark contrast to the traditional three-way stopcock wherein the channel in the rotating cam restricts the flow path.

Alternatively, an additional component in this valve embodiment may comprise a lock that engages with the shell to keep the valve in a depressed position when the external pressure is released. This lock may then be actuated to lock to close the lumen of the elongate member and subsequently disengaged to release in order to allow the valve to return to its original position. The locking and unlocking of this embodiment is at the discretion of the user. Examples of such locks include a detent, a latch, or the like.

In an alternate embodiment, the valve may also comprise a feature that interacts with the cylindrical opening, for example, a keyway and a key, or a tongue and a groove, that maintain their axial orientation and prevents the valve from rotating within the cylindrical opening. The valves may be fabricated from materials known to the art including, but not limited to polycarbonate, polyethylene, polyolefin, polypropylene, polytetrafluoroethylene, polysulfone, polyvinylchloride, polyoxymethylene (Delrin®), brass, stainless steel, nylon, perfluoroalkoxy (e.g., Teflon®), natural rubber, nitrile rubber, silicone rubber, combinations thereof, and the like.

The external surface of the disk may further comprise features including, but not limited to, concave areas, convex areas, areas comprising a high-grip texture or textures, relatively high friction pads (e.g., silicone or polyurethane pads), raised or lowered areas, combinations thereof, and the like. The valve may be fabricated in a different color from the housing of the device, and different valves on a single device may be fabricated in different colors. Further, tactile features such as raised dots may be used to differentiate one valve from another on a single device as a means to aid patients that may have poor vision or color-blindness.

In another example, the previously described valve may further comprise a spring coiled around the pillar. One end of the spring may rest against the underside of the disk with the other end of the spring resting against a flange that extends from the interior surface of the housing and is positioned over the top of the elongate member. The flange further comprises a circular hole concentrically oriented with the pillar. The diameter of the hole is larger than the pillar of the valve and smaller than that of the spring, allowing the pillar to pass through the flange and interact with the elongate member.

As the patient applies pressure to the top surface of the disk and advances the valve towards the elongate member, the spring compresses. When the patient stops depressing the valve, the spring resiliently expands and pushes the valve back into its starting position. Features that assist in returning the valve to the starting position may be useful when the elasticity, wall thickness, diameter, and/or other characteristic of the elongate member is such that it cannot provide enough force to fully return the valve to its initial position. Other components for assisting the return of the valve may be used as are known to the art including, but not limited to, leaf springs, non-coiled springs, cantilevered springs, wave springs, air-filled bladders, elastic rings, elastic bands, and the like.

An alternative design of the valve has a general shape as described earlier for the disk and pillar valve, but further comprises external threads on the outer circumferential surface of the disk. In this design, complementary internal threads are located on the inner wall of the openings in the housing. The set of threads may be right-handed or left-handed. In the housing of right-handed threads, turning the disk in a clockwise manner advances the valve towards the elongate member and eventually closes the flow path. Turning the disk in a counter-clockwise manner raises the valve and opens the elongate member. The number of turns needed to close or open the elongate member is dictated by the thread pitch, relative coarseness or fineness of the thread, and the length of travel between the open and closed positions of the valve, e.g., may only require a partial turn, one full turn, or multiple turns, as desired.

The threaded valve may comprise additional features that assist the patient in turning the valve. These features may include, but are not limited to, extruded shapes on the external surface of the disk such as wings, bars, and the like that provide an enhanced grip on the valve, recessed features in the external surface of the disk such as channels, semicircles, and the like that do the same, inscriptions, printing, labels and the like that provide the patient information (such as what direction to turn the valve), combinations thereof, and the like. One advantage of this design is that constant pressure is not needed to maintain closure of the elongate member, nor is there a risk of accidental closure of the elongate member due to inadvertent pressure on the valve.

Another alternative design of the valve is that of an intravenous (IV) roller clamp, wherein the housing of the device comprises the ramp and slot features of an IV roller clamp, e.g., where the valve is the ridged wheel of an IV roller clamp, and the elongate member is a corollary to the IV line. As the ridged wheel is rolled towards the shallow end of the ramp, the wheel compresses and closes the elongate member. To open the elongate member, the ridged wheel is rolled in the opposite direction (to the deeper end of the ramp). This design also has the advantages of not requiring constant pressure to maintain closure of the elongate member and minimizing the risk of accidental closure of the elongate member due to inadvertent pressure on the valve.

Yet another design of the valve is system including a living hinge and a detent. The living hinge and detent may be an integral feature of the housing, or alternatively, may be a distinct assembly joined to the housing using means known in the art including, but not limited to, adhesive bonding, welding, ultrasonic welding, over-molding, threading and tapping, mechanical fixation, friction or interference fits, combinations thereof, and the like. When the elongate member is in the open configuration, the free end of the living hinge rests on or above the wall of the elongate member. At least one detent is positioned above the elongate member and oriented such that the detent will reversibly hold the living hinge in a static position once the free end of the hinge passes the detent. For example, the detent may be fabricated from a relatively flexible material that is shaped and oriented such that the detent flexes away from the free end of the hinge as the hinge is depressed, then returns to its original position once the free end of the hinge passes below the detent. To close the lumen of the elongate member, the user advances the free end of the living hinge towards the elongate member until the lumen is closed and the detent locks the living hinge in position. To return the living hinge to its original position and open the lumen of the elongate member, the detent is pushed away from the free end of the living hinge. While the system described herein comprises components, it should be clear to one of skill in the art that a living hinge and detent system may comprise additional components and designs.

Another alternative design of the valve is that of a rocker switch. In the simplest embodiment, the rocker switch comprises a relatively rigid bar with a cylindrical hole running through the short axis of the bar at the midpoint of the bar and substantially perpendicular to the long axis of the bar. An axle passes through the hole, allowing the bar to rotate freely about the axle. The axle has a length that enables portions of the axle to protrude beyond the bar when the bar is centered on the midpoint of the axle. The exposed portions of the axle are mounted into the housing such that each end of the bar is located above one of the two elongate members.

For example, a housing may be provided that includes two complementary shells with bore-holes present at equivalent positions in each shell that are sized to provide an interference fit with the axle. The axle may be pressed into one of the bore-holes, the bar placed over the axle, and the second shell pressed onto the exposed portion of the axle (along with other pins and/or bore holes on the two shells mating the hold the shells together). The bar is then free to rotate within the constraints presented by the geometry of the housing, the position of the elongate members and other components of the device, and the dimensions of the bar.

To close the lumen of one of the two elongate members, the end of the bar above the elongate member of interest is depressed until the lumen of the elongate member is closed. To open a closed lumen, the end of the bar opposite the elongate member of interest is depressed. Other embodiments of the rocker switch design may include detents to hold the rocker switch in position once depressed, springs or other means of modulating the stroke or travel of the ends of the rocker, ratcheting arrangements, and the like.

Any of the embodiments described herein may further include one or more additional components, such as one or more flow or volume sensors, transmission apparatus, power sources, audible, tactile, or visual indicators relating the state of a given elongate member (e.g., open or closed), integrated circuits, and the like. For example, a device including a power source, flow sensors, an integrated circuit, and transmission apparatus may record the amount and/or rate of material being drained from the patient and transmit that data to a remote display, such as a smartphone, tablet, personal computer, wearable display device, cloud-based medical service, physician's office, or the like.

Alternatively, a device including a power source, LED, electrical wiring, and strike plates may provide a visual signal (e.g., the LED activating) to the patient when a valve has been depressed to a degree that allows the strike plates to connect and complete an electrical circuit between the LED and power source. Other electrical components, such as resistors, capacitors, and the like needed for implementation of such a design would be incorporated into the device.

Similarly, the use of an audible signal, such as a tone, may be employed in place of or in combination with a visual signal to indicate restriction or closure of the lumen of an elongate member. For example, the source of the audible signal may be a powered speaker or may be a "click" produced by the interference of two or more parts of the device (e.g., a flange on a valve and a second flange attached to or integral to the housing). Another source of the audible signal contemplated herein comprises of a convex sheet metal component that emits a sound when force is applied to reconfigure the component to a concave condition. When the force is removed, the component may resiliently revert to its original convex condition based upon the spring characteristic of the component and its sheet metal material. The component may also emit another sound as it returns to the convex condition. Such a component may be positioned in the shell about the valve and where it can be actuated by the valve.

The device may be used to alternately enable drainage, infusion, or instillation of an abscess or other physiological structure and flush the drainage, infusion, or instillation catheter and/or line and vacuum line of the broader drainage, infusion or instillation system. In this example, the device includes two elongate members (one with a male luer-lock coupling, the other with a female luer-lock coupling), a connector, a luer-activated valve directly attached to the connector, a housing, and two valves (one placed over each elongate member). For example, when orienting the components from left to right, the arrangement of the internal components of the device is as follows: male luer-lock coupling, elongate member, connector, elongate member, female luer lock coupling. This arrangement may be linear and lie along a single horizontal axis. The lumens of the connector are in a "T" shape, and the outlet of the luer-activated valve is directly joined to the vertical lumen of the connector. The valves of this example are disk-and-pillar valves as previously described.

The drainage, infusion, or instillation system may also include a drainage, infusion, or instillation catheter having a proximal end directly connected to the device (or alternatively connected using a catheter line connecting the drainage, infusion or instillation catheter to the male luer-lock coupling of the device), a vacuum line connected to the female luer-lock coupling of the device, a vacuum source, and an infusion syringe. In this example, the vacuum source may be provided by a rubber suction bulb, however, it should be clear that other vacuum sources are contemplated. To apply negative pressure to the drainage, infusion, or instillation catheter, the suction bulb is compressed and connected to the vacuum line in the compressed state. Once connected to the drainage, infusion, or instillation system, the suction bulb is released and the negative pressure draws fluid through the catheter, catheter line, device, vacuum line, and into the suction bulb for collection.

To flush the vacuum line, the valve closest to the drainage, infusion or instillation line is depressed to block flow to the drainage, infusion, or instillation line, and an infusion syringe containing the flushing media is attached to the inlet of the luer-activated valve. The connection of the infusion syringe to the inlet of the luer-activated valve opens a flow path from the syringe to the vacuum line. The infusion media is then flushed through the system from the luer-activated valve to the vacuum line. The infusion syringe is removed when the flushing operation is complete (closing the flow path through the luer-activated valve) and the valve returns to its original position opening the flow path to the drainage, infusion or instillation line.

The same process may then be repeated for the drainage, infusion, or instillation catheter. The valve closest to the vacuum line is depressed to block flow to the vacuum line, the infusion syringe is connected to the luer-activated valve, the drainage, infusion or instillation catheter is flushed with infusion media, the infusion syringe is removed and the valve returns to its original position to open the flow path. If prescribed, the drainage, infusion, or instillation catheter or wound itself may be irrigated or flushed in the same manner by disconnecting the drainage or infusion/instillation catheter line from the drainage or infusion/instillation catheter.

Other aspects and features of the present invention will become more apparent from consideration of the following description taken in conjunction with the accompanying drawings.

### BRIEF DESCRIPTION OF THE DRAWINGS:

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures.
FIGS. 1A through ID are schematic illustrations of the different states of flow enabled by an exemplary embodiment of a valve device.
FIG. 2 is an exploded view of an exemplary embodiment of a valve device.
FIG. 3 is an isometric view of the embodiment shown in FIG. 2.
FIGS. 4A and 4B are cross-sectional views of an exemplary embodiment of a valve member that may be included in a valve device, such as that shown in FIG. 2.
FIG. 5 is a cross-sectional view of an alternative embodiment of a valve member that may be included in a valve device, such as that shown in FIG. 2.
FIG. 6A through 6C are cross-sectional views of alternative embodiments of valve members that may be included in a valve device, such as that shown in FIG. 2.
FIG. 7 is a cross-sectional view of an alternative embodiment of a valve member that may be included in a valve device, such as that shown in FIG. 2.

### DETAILED DESCRIPTION OF THE EXEMPLARY EMBODIMENTS:

Before the exemplary embodiments are described, it is to be understood that this invention is not limited to particular embodiments described, as such may, of course, vary. It is also to be understood that the terminology used herein is for the purpose of describing particular embodiments only, and is not intended to be limiting, since the scope will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the recited range. The upper and lower limits of these smaller ranges may independently be included or excluded in the range, and each range where either, neither, or both limits are included in the smaller ranges is also encompassed within the recited range, subject to any specifically excluded limit in the stated range. Where the stated range includes one or both of the limits, ranges excluding either or both of those included limits are also included in the recited range.

Unless defined otherwise, all technical and scientific terms used herein have the same meaning as commonly understood by one of ordinary skill in the art. Although any methods and materials similar or equivalent to those described herein can be used in the practice or testing of the embodiments described, some potential and preferred methods and materials are now described.

It must be noted that as used herein and in the appended claims, the singular forms "a," "an," and "the" include plural referents unless the context clearly dictates otherwise. Thus, for example, reference to "a compound" includes a plurality of such compounds and reference to "the polymer" includes reference to one or more polymer and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

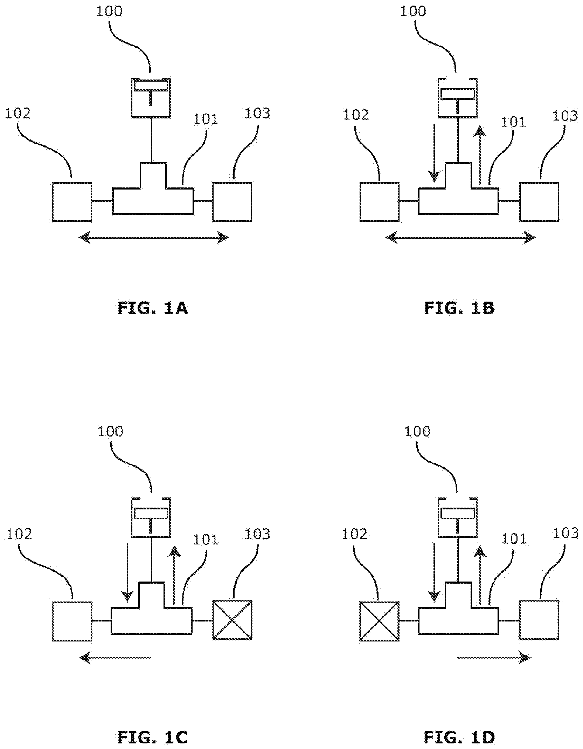

FIGS. 1A through 1D are schematic illustrations of the flow paths through the internal components of an exemplary device when the device is in one of four states. The device is arranged identically in each of the figures and includes the following components: a connector 101 joined to a luer-activated valve 100 and to elongate tubular members 102 and 103. FIG. 1A illustrates the device at baseline, when no luer tipped coupling (e.g., a standard luer lock or slip luer syringe tip, not shown) is connected to the luer-activated valve 100. In this state, flow (as shown by the arrows) is diverted between elongate tubular member 102 and elongate tubular member 103 in both directions and flow is restricted between any of the components and the luer-activated valve 101.

FIG. 1B illustrates the device once a luer-tipped coupling is inserted into and/or otherwise coupled to the port of the luer-activated valve 100. In this state, flow is diverted between all components in all directions. FIG. 1C illustrates the flow paths present in the device when a luer-tipped coupling is inserted into and/or otherwise coupled to the inlet of the luer-activated valve 100 and the elongate member 103 is closed off, blocking flow through elongate member 103. In this state, bi-directional flow is diverted between elongate member 102 and luer-activated valve 100.

FIG. 1D illustrates the flow paths present in the device when a luer-tipped coupling is inserted into and/or otherwise coupled to the inlet of the luer-activated valve 100 and the elongate member 102 is closed off, blocking flow through elongate member 102. In this state, bi-directional flow is diverted between elongate member 103 and luer-activated valve 100.

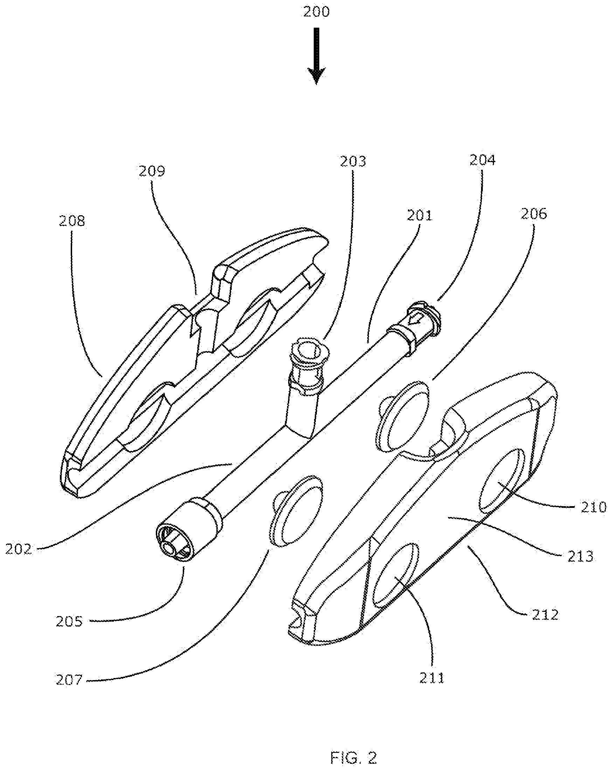

FIG. 2 is an exploded view of an exemplary embodiment of a valve device 200 including two elongate members 201 and 202, a luer-activated valve 203, fittings 204 and 205, valves 206 and 207, and a housing including a pair of shells 208 and 212. A connector joining elongate member 201, elongate member 202, and luer-activated valve 203 is not shown for clarity. The elongate members 201 and 202 each includes a lumen that is in communication with the respective lumens of luer-activated valve 203 and fittings 204 and 205. In an exemplary embodiment, the elongate members 201 and 202 may be constructed using relatively elastic or compliant materials, e.g., as described elsewhere herein. Further, the design of elongate members 201 and 202 and the connector (not shown) is such that the size (i.e., inner diameter) of the respective lumens of these components is generally constant along the length of the component.

In this embodiment, the fitting 204 may be a female luer-lock coupling and the fitting 205 may be a male luer-lock coupling. The inlet of the luer-activated valve 203 may be a female luer-lock coupling. While this embodiment includes specific couplings for the fittings 204 and 205, and for the inlet of luer-activated valve 203, it should be clear to one of skill in the art that many types and designs of couplings are suitable for use in the valve devices herein, including but not limited to the examples listed previously.

For example, if a high flow rate through the fitting 205, elongate member 202, connector (not shown), elongate member 201, and fitting 204 is desired, the fittings 204 and 205 may include externally threaded couplings that do not include the reduction in inner diameter associated with a mated pair of standard luer couplings. Valves 206 and 207 may be of the disk and pillar design previously described, and are aligned substantially perpendicular to and centered over elongate members 201 and 202, respectively.

The shells 208 and 212 may enclose the assembly of the elongate members 201 and 202, the luer-activated valve 203, the fittings 204 and 205, and the connecter (not shown), along with valves 206 and 207. The external faces of the shells 208 and 212 feature relatively flat and smooth surface and the edges rounded, filleted or contoured to enhance patient comfort during use. This may be useful in cases where the device is in body position whereby the user may be lying or sitting over the device.

In this exemplary scenario, the external surface of the device that is in direct contact with the user does not present discomfort to the user such as pinching or poking of the skin or tissue, which may otherwise potentially result in trauma, irritation, or injury, such as abrasion, cut, bruising, ulceration, and the like. Optionally, the shell 208 may further include a cutout 209 that provides a pocket or cavity in which the inlet of luer-activated valve 203 can reside. The cutout 209 may serve to protect the inlet of luer-activated valve 203 from breakage due to accidental contact, stress, or strain during use, as well as to provide comfort to the user by hiding the sharp edges or corners of the luer-activated valve 203.

The shell 212 may further include two openings 210 and 211 that are centrally aligned along and over elongate members 201 and 202, respectively. The openings 210 and 211 may be of a diameter that is larger than the diameter of the disk component of the valves 206 and 207. It is further contemplated that the diameter of the openings 210 and 211 may be optimized to accommodate access to the valves 206 and 207 via a user's fingers, for example with a diameter between about 0.25 inch and 1.0 inch (6.25-25.4 mm), or between about 0.5 inch and 0.75 inch (12.5-18.75 mm).

Optionally, the shells 208 and 212 may be fabricated to include a second material external to the outer surface of the shells 208 and 212 that is softer in property (not shown). The materials may include, but not limited to silicone, urethane, and the like, e.g., as previously discussed. These types of material may enhance patient comfort when the device is in direct contact with the tissue or skin. Further, it may provide a user with a more stable, gripping surface. The second softer material may be applied to the shells 208 and 212 using processes known in the art such as over-molding, coating, or the like.

While the embodiment shown in FIG. 2 is described as comprising a connector (not shown in the figure) that joins the elongate member 201, elongate member 202, and luer-activated valve 203, it should be clear to one of skill in the art that the elongate member 201, elongate member 202, and luer-activated valve 203 may be joined directly to each other, e.g., using methods known to the art, including but not limited to, bonding, welding, ultrasonic welding, over-molding, threading/tapping, crimping, combinations thereof, and the like.

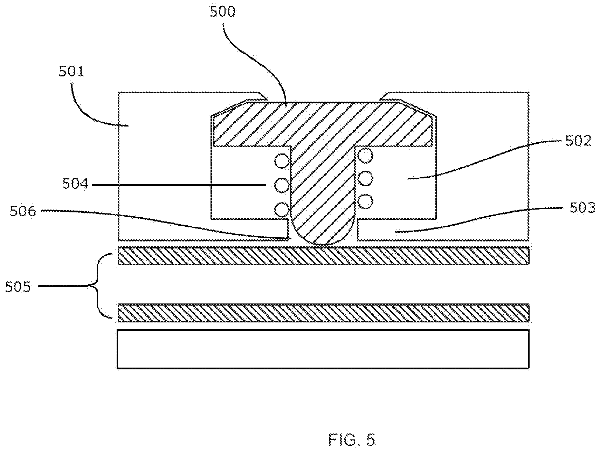

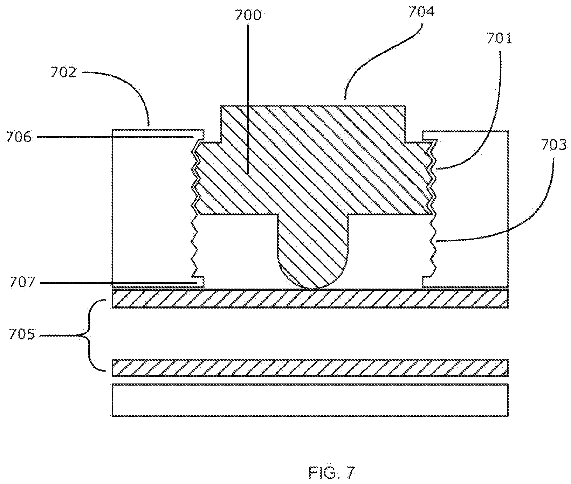

Furthermore, the elongate members 201 and 202 may be fabricated out of a single length of tubing, and joined to luer-activated valve 203 using methods known to the art, including but not limited to bonding, welding, ultrasonic welding, over-molding, threading/tapping, crimping, combinations thereof, and the like. Furthermore, while the valves 206 and 207 are shown to be of the same design, it should be clear to one of skill in the art that each valve may have an independent design. For example, in an alternative embodiment of the valve device, the valve 206 may be externally threaded (e.g., as shown in FIG. 7) with complementary internal threads located about opening 210, while the valve 207 may further comprise a spring (e.g., as shown in FIG. 5) to assist the return stroke of the valve to its baseline state.

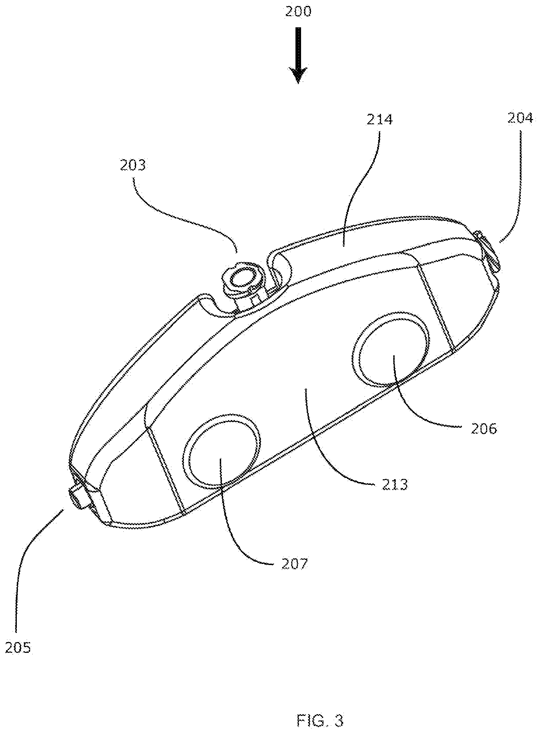

FIG. 3 is an isometric view of the components of an embodiment of the valve device 200 assembled together into a single unit. The inlet of luer-activated valve 203 and the couplings of fittings 204 and 205 are substantially inset into the housing 214 (comprising shells 208 and 212 shown in FIG. 2). The valves 206 and 207 are coaxially aligned with the openings 210 and 211 (shown in FIG. 2) and substantially flush with surface 213 of the housing 214.

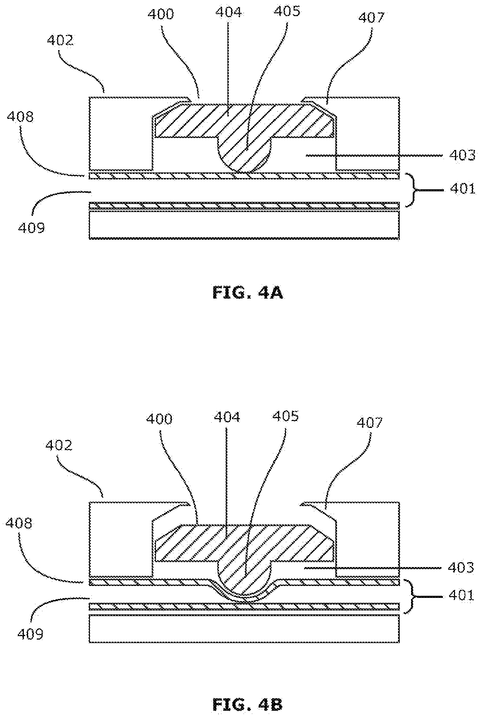

FIGS. 4A and 4B show a cross sectional view of an exemplary configuration of a valve 400 and an elongate member 401 when the elongate member 401 is in the open and closed states. FIG. 4A shows valve 400 slidably disposed within opening 403 of shell 402. The valve 400 may further include a disk 404 and a pillar 405. The disk 404 may be tapered to mate with rim 407 located near the surface of the shell 402. The lower end of the pillar 405 may be rounded into a hemispherical shape. Alternatively, other shapes can be employed at the tip of the pillar 405 (i.e., where the pillar 405 makes contact with the elongate member 401), which may provide effective closing of the lumen 409 of the elongate member 401 such as a wedge, a fin, or the like.

The rim 407 may be oriented such that it is raised or elevated and adjoined from the external surface of the shell 402 and positions the valve 400 slightly recessed from the surface of the shell 402. The recessed configuration of the valve 400 may prevent the valve 400 from being depressed accidentally, for example, in cases where the device is in a body position where the outer face of the shell 402 is resting against a surface (i.e., skin/soft tissue or a pillow), which can potentially push the valve 400 and effectively close the lumen 409 of the elongate member 401.

The opening 403 may be of cylindrical shape and may further include one or more features, such as a keyway or a groove (not shown), positioned along the wall of the opening 403, whereby the valve 400 has a matching key or protrusion (not shown) that engages with the keyway or groove, and prevents the valve 400 from rotating within the cylindrical opening 403. Incorporation of this feature may be useful when the pillar 405 of the valve 400 is shaped to a wedge or a fin (not shown) by keeping the orientation or position of the wedge or fin (not shown) relative to the elongate member 401.

The elongate member 401 may further include a wall 408 and a lumen 409. In FIG. 4A, the valve 400 is in the baseline state wherein the lumen of the elongate member 401 is open and the disk 404 of the valve 400 is substantially flush with the rim 407 of the shell 402. In FIG. 4B, the valve 400 is in an active state wherein the valve 400 has been depressed within the opening 403 towards the elongate member 401 to a degree such that the tip of the pillar 405 has compressed the wall 408 of the elongate member 401 and closed the lumen 409.

In this valve configuration, the material used for the elongate member 401 may have a compliant or elastic property so that the wall 408 can provide sufficient spring force to push back and return the valve 400 to its original baseline state as soon as the external pressure applied to the valve 400 is released. The thickness of the wall 408 may also be varied along the length of the elongate member 401. In particular, the thickness of the wall 408 that is in alignment with the pillar 405 may be used to tune or define the spring force provided by the wall 408 in order to most effectively return the valve 400 to its original baseline state.

FIG. 5 shows a cross section view of an alternative embodiment of a valve device including a valve 500, shell 501, spring 504, and elongate member 505. The shell 501 further includes an opening 502 and a flange 503. The flange 503 further includes a flange opening 506. The spring 504 is disposed about the valve 500 and between the lower surface of the valve 500 and the upper surface of the flange 503. The flange opening 506 is sized to allow the pillar of the valve 500 to move freely within the flange opening 506 while preventing movement of the lower end of the spring 504. The valve 500 is coaxially aligned with the opening 502 and the flange opening 506.

Advancement of the valve 500 towards the elongate member 505 compresses the spring 504. When downward pressure on the valve 500 is released, the spring 504 applies force to the valve 500 and aids in returning the valve 500 to its initial baseline position within the opening 502. It should be clear to one of skill in the art that the spring 504 may comprise various lengths, widths, coil thicknesses, spring constants, and the like as needed to achieve desired functional characteristics. For example, a spring length may be chosen that is greater than the distance between the flange 503 and the underside of the disk of the valve 500. This would result in the spring 504 providing pressure against the valve 500 in the baseline condition (i.e., with no external pressure applied to the valve 500), and maintain the valve 500 in a position against the underside of the shell 501.

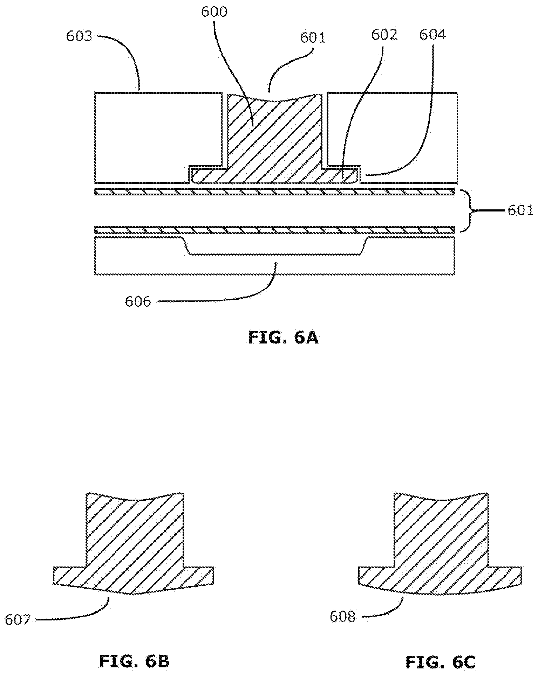

FIGS. 6A through 6C show cross sectional views of alternative embodiments of a valve device. FIG. 6A shows a valve 600 including a concave external surface 601 and a flange 602 slidably disposed within the shell 603. The shell 603 includes an undercut 604 and a receiving cavity 606. The interaction between the flange 602 and the undercut 604 prevents the surface 601 of the valve 600 from extending beyond the exterior of the shell 601. The receiving cavity 606 enables the elongate member 601 to more easily compress as the valve 600 is slidably translated towards the elongate member 601. As can be seen in FIGS. 6B and 6C, the lower face of the valve 600 may include a number of different contours, including but not limited to conical 607 and/or rounded 608. Likewise (not shown), the external surface of the valve 600 may have varied shapes, textures, and/or feature that are beyond those shown in this example.

FIG. 7 is a cross sectional view of yet another alternative embodiment of a valve device including a turn valve 700. The turn valve 700 includes external threads 701 that mate with internal threads 703 of shell 702. The turn valve 700 further includes a raised feature 704 that allows the patient to grasp and turn the turn valve 700 relative to the shell 702. In a housing where the threads 701 and 703 are right-handed, turning the turn valve 700 clockwise will advance the turn valve 700 towards the elongate member 705. Turning the turn valve 700 counter-clockwise will retract the turn valve 700 back towards the surface of the shell 702. In a housing where the threads 701 and 703 are left-handed, the turn valve 700 will respond in the opposite manner to clockwise or counter-clockwise rotation. Rims 706 and 707 in the shell 702 prevent the turn valve 700 from advancing too far in either direction.

During use, any of the devices herein may be used to drain fluids from and/or infuse fluids into a patient's body, e.g., via a catheter or other tubular member (not shown) at least partially implanted in the patient's body. For example, the catheter may include a first end implanted at or otherwise introduced into a surgical site, treatment site, or other location in the patient's body such that a second end of the catheter extends extracorporeally from the patient's body. The second end of the catheter may be coupled to one of the connectors of a valve device, such as the first connector 205 of the valve device 200 shown in FIG. 2. A container, e.g., bag, may be provided that includes tubing that may be coupled to another of the connectors of the valve device, such as the second connector 204 shown in FIG. 2. Once the catheter and container are coupled to the valve device 200, the primary fluid path through the elongate members 201, 202 allows fluid from the patient's body to be drained through the catheter into the bag, as described elsewhere herein. Alternatively, a container with a vacuum capability (e.g., a device commonly used in the field called Jackson Pratt) may be used to provide active suction of the fluid or other drainage materials in place of a passive container.

Because the third connector 203 is a luer-activated connector, it is configured to prevent fluid flow therethrough unless a mating connector is fully coupled thereto. Thus, the secondary fluid path between the elongate members 201, 202 and the third connector 203 remains closed, thereby preventing fluid from flowing other than between the catheter and the container.

At any time, a source of at least one of vacuum and infusion media, e.g., a suction ball, syringe, and the like (not shown) may be coupled to the third connector 203 to open the secondary flow path between the primary fluid path and the source. At least one of the first and second valve members 206, 207 may be activated to selectively close at least a portion of the primary fluid path. For example, the first valve member 206 may be activated to prevent fluid flow to the container, and the source may be activated, e.g., to aspirate fluid through the primary fluid path and the tubing to the catheter and/or infuse fluid into the primary fluid path and the catheter. Alternatively, the second valve member 207 may be activated to prevent fluid flow to the catheter, and the source may be activated, e.g., to aspirate fluid through the primary fluid path and the tubing to the container and/or infuse fluid into the primary fluid path and the tubing, as desired, e.g., to clear debris or otherwise enhance subsequent drainage and/or treatment of the patient.

In some embodiments of a valve device according to the present invention, the first valve member may be biased to the first position to keep flow through the primary fluid path open when the first valve member is not intentionally directed to the second position.

In some embodiments of a valve device according to the present invention, the second valve member may be biased to the first position to keep flow through the primary fluid path open when the second valve member is not intentionally directed to the second position.

In some embodiments of a system according to the present invention, the source of infusion media may comprise a syringe comprising an infusion media connector configured to couple to the third connector.

In some embodiments of a system according to the present invention, the first valve member may be movable axially within the housing between a first position in which the first valve member does not obstruct the primary fluid path and a second position in which the primary fluid path is closed to prevent fluid flow through the first connector; and the second valve member may be movable axially within the housing between a first position in which the second valve member does not obstruct the primary fluid path and a second position in which the primary fluid path is closed to prevent fluid flow through the second connector.

In some embodiments of a system according to the present invention, the first and second valve members may be biased to the first position.

In some embodiments of a system according to the present invention, the first and second valve members may be biased to the first position to keep flow through the primary fluid path open when the first and second valve members are not intentionally directed to the second position.

In some embodiments of a system according to the present invention, with the first valve member in the first position and the second valve member in the second position, an open fluid path may be provided between the first and third connectors.

In some embodiments of a system according to the present invention, with the first valve member in the second position and the second valve member in the first position, an open fluid path may be provided between the second and third connectors.

In some embodiments of a system according to the present invention, the housing may comprise a recess and wherein the third connector may be disposed within the recess.

## Claims

1. A valve device (200) for draining fluids from and/or infusing fluids into a patient's body, comprising:
a housing (214) comprising first, second, and third connectors (205; 204; 203), and a primary fluid path communicating between the first and second connectors (205; 204) and a secondary fluid path communicating from the primary fluid path to the third connector (203), wherein the third connector (203) is configured to prevent fluid flow therethrough unless a mating connector is fully coupled thereto;
a first valve member (206) movable within the housing (214) between a first position in which the first valve member (206) does not obstruct the primary fluid path and a second position in which the primary fluid path is closed to prevent fluid flow through the first connector (205); and
a second valve member (207) movable within the housing (214) between a first position in which the second valve member (207) does not obstruct the primary fluid path and a second position in which the primary fluid path is closed to prevent fluid flow through the second connector (204),
wherein the secondary fluid path is disposed between the first valve member (206) and the second valve member (207).

2. The valve device (200) of claim 1, wherein the first valve member (206) is axially movable within the housing (214) between the first position and the second position.

3. The valve device (200) of claim 1 or claim 2, wherein the second valve member (207) is axially movable within the housing (214) between the first position and the second position.

4. The valve device (200) of any one of claim 1-3, wherein, with the first valve member (206) in the first position and the second valve member (207) in the second position, an open fluid path is provided between the first and third connectors (205; 203).

5. The valve device (200) of any one claim 1-4, wherein, with the first valve member (206) in the second position and the second valve member (207) in the first position, an open fluid path is provided between the second and third connectors (204; 203).

6. The valve device (200) of any one of claim 1-5, wherein the housing (214) comprises a recess and wherein the third connector (203) is disposed within the recess.

7. The valve device (200) of any one of claim 1-6, wherein the third connector (203) is a luer-activated connector.

8. The valve device (200) of any one of claim 1-7,
wherein the first and second valve members (206; 207) are recessed within the housing (214) and configured to prevent accidental closure or restriction of the primary fluid path.

9. A system for draining fluids from and/or infusing fluids into a patient's body, comprising:
a valve device (200) according to claim 1;
a tubular member (102, 103) comprising a first end sized for introduction in a patient's body and a second end comprising a tubular member connector configured to couple to the first connector (205); and
a container for storing fluid from the patient's body comprising tubing including a container connector configured to couple to the second connector (204), thereby providing a fluid path from the tubular member through the primary fluid path and tubing into the container.

10. The system of claim 9, further comprising a source of vacuum comprising a vacuum connector configured to couple to the third connector (203), whereupon the secondary flow path is opened to provide a fluid path between the primary fluid path and the source of vacuum.

11. The system of claim 10, wherein the source of vacuum comprises a syringe.

12. The system of claim 9, further comprising a source of infusion media configured to couple to the third connector, whereupon the secondary flow path is opened to provide a fluid path between the primary fluid path and the source of infusion media.

## Patentansprüche

1. Eine Ventilvorrichtung (200) zum Ablassen von Fluiden aus dem Körper eines Patienten und/oder zur Infusion von Fluiden in den Körper des Patienten, aufweisend
ein Gehäuse (214) aufweisend einen ersten, zweiten und dritten Anschluss (205; 204; 203) und einem primären Fluidpfad, der fluidleitend zwischen dem ersten und zweiten Anschluss (205; 204) ist, und einem sekundären Fluidpfad, der fluidleitend von dem primären Fluidpfad zu dem dritten Anschluss (203) ist, wobei der dritte Anschluss (203) eingerichtet ist, einen Fluidfluss durch sich zu verhindern, solange kein passender Anschluss vollständig daran gekoppelt ist;
ein erstes Ventilelement (206), das innerhalb des Gehäuses (214) beweglich ist zwischen einer ersten Position, in der das erste Ventilelement (206) den primären Fluidpfad nicht versperrt, und einer zweiten Position, in der der primäre Fluidpfad geschlossen ist, um einen Fluidstrom durch den ersten Anschluss (205) zu verhindern; und
ein zweites Ventilelement (207), das innerhalb des Gehäuses (214) beweglich ist zwischen einer ersten Position, in der das zweite Ventilelement (207) den primären Fluidpfad nicht versperrt, und einer zweiten Position, in der der primäre Fluidpfad geschlossen ist, um einen Fluidstrom durch den zweiten Anschluss (204) zu verhindern,
wobei der sekundäre Fluidpfad zwischen dem ersten Ventilelement (206) und dem zweiten Ventilelement (207) angeordnet ist.

2. Ventilvorrichtung (200) nach Anspruch 1, wobei das erste Ventilelement (206) innerhalb des Gehäuses (214) zwischen der ersten Position und der zweiten Position axial beweglich ist.

3. Ventilvorrichtung (200) nach Anspruch 1 oder Anspruch 2, wobei das zweite Ventilelement (207) innerhalb des Gehäuses (214) zwischen der ersten Position und der zweiten Position axial beweglich ist.

4. Ventilvorrichtung (200) nach einem der Ansprüche 1-3, wobei mit dem ersten Ventilelement (206) in der ersten Position und dem zweiten Ventilelement (207) in der zweiten Position ein offener Fluidpfad zwischen dem ersten und dritten Anschluss (205; 203) bereitgestellt ist.

5. Die Ventilvorrichtung (200) nach einem der Ansprüche 1-4, wobei mit dem ersten Ventilelement (206) in der zweiten Position und dem zweiten Ventilelement (207) in der ersten Position ein offener Fluidpfad zwischen dem zweiten und dritten Anschluss (204; 203) vorgesehen ist.

6. Die Ventilvorrichtung (200) nach einem der Ansprüche 1-5, wobei das Gehäuse (214) eine Aussparung aufweist und wobei der dritte Anschluss (203) innerhalb der Aussparung angeordnet ist.

7. Ventilvorrichtung (200) nach einem der Ansprüche 1-6, wobei der dritte Anschluss (203) ein Luer-aktivierter Anschluss ist.

8. Die Ventilvorrichtung (200) nach einem der Ansprüche 1-7,
wobei das erste und das zweite Ventilelement (206; 207) in das Gehäuse (214) eingelassen und so eingerichtet sind, dass ein versehentliches Schließen oder Verengen des primären Fluidpfades verhindert wird.

9. Ein System zur Ableitung von Fluid aus dem Körper eines Patienten und/oder zur Infusion von Fluid in den Körper des Patienten, aufweisend:
eine Ventilvorrichtung (200) nach Anspruch 1;
ein röhrenförmiges Element (102, 103) aufweisend ein erstes Ende, das für die Einführung in den Körper eines Patienten bemessen ist, und ein zweites Ende aufweisend einem röhrenförmigen Elementanschluss, der geeignet ist, mit dem ersten Anschluss (205) gekoppelt zu werden; und
einen Behälter zum Aufbewahren von Fluid aus dem Körper des Patienten, der eine Rohrleitung mit einem Behälteranschluss aufweist, der geeignet ist, an den zweiten Anschluss (204) gekoppelt zu werden, wodurch ein Fluidpfad von dem röhrenförmigen Element durch den primären Fluidpfad und die Rohrleitung in den Behälter bereitgestellt wird.

10. Das System nach Anspruch 9 ferner aufweisend eine Vakuumquelle, aufweisend einen Vakuumanschluss, der geeignet ist, an den dritten Anschluss (203) gekoppelt zu werden, woraufhin der sekundäre Strömungspfad geöffnet wird, um einen Fluidpfad zwischen dem primären Fluidpfad und der Vakuumquelle bereit zu stellen.

11. Das System nach Anspruch 10, wobei die Vakuumquelle eine Spritze aufweist.

12. Das System nach Anspruch 9 ferner aufweisend eine Quelle für Infusionsmedien, die geeignet sind, an den dritten Anschluss gekoppelt zu werden, woraufhin der sekundäre Strömungspfad geöffnet wird, um einen Fluidpfad zwischen dem primären Fluidpfad und der Quelle für Infusionsmedien zu schaffen.

## Revendications

1. Dispositif de valve (200) pour drainer des fluides à partir d'un corps d'un patient et/ou pour perfuser des fluides dans le corps du patient, comprenant :
un logement (214) comprenant des premier, deuxième, et troisième raccords (205 ; 204 ; 203), et un trajet de fluide principal communiquant entre les premier et deuxième raccords (205 ; 204) et un trajet de fluide secondaire communiquant à partir du trajet de fluide principal vers le troisième raccord (203), dans lequel le troisième raccord (203) est configuré pour empêcher le fluide de s'écouler à travers celui-ci sauf si un raccord de couplage est entièrement couplé à celui-ci ;
un premier élément de valve (206) mobile dans le logement (214) entre une première position dans laquelle le premier élément de valve (206) n'obstrue pas le trajet de fluide principal et une deuxième position dans laquelle le trajet de fluide principal est fermé pour empêcher le fluide de s'écouler à travers le premier raccord (205) ; et
un deuxième élément de valve (207) mobile dans le logement (214) entre une première position dans laquelle le deuxième élément de valve (207) n'obstrue pas le trajet de fluide principal et une deuxième position dans laquelle le trajet de fluide principal est fermé pour empêcher le fluide de s'écouler à travers le deuxième raccord (204),
dans lequel le trajet de fluide secondaire est disposé entre le premier élément de valve (206) et le deuxième élément de valve (207).

2. Dispositif de valve (200) selon la revendication 1, dans lequel le premier élément de valve (206) est mobile axialement dans le logement (214) entre la première position et la deuxième position.

3. Dispositif de valve (200) selon la revendication 1 ou la revendication 2, dans lequel le deuxième élément de valve (207) est mobile axialement dans le logement (214) entre la première position et la deuxième position.

4. Dispositif de valve (200) selon l'une quelconque des revendications 1-3, dans lequel, avec le premier élément de valve (206) dans la première position et le deuxième élément de valve (207) dans la deuxième position, un trajet de fluide ouvert est fourni entre les premier et troisième raccords (205 ; 203).

5. Dispositif de valve (200) selon l'une quelconque des revendications 1-4, dans lequel, avec le premier élément de valve (206) dans la deuxième position et le deuxième élément de valve (207) dans la première position, un trajet de fluide ouvert est fourni entre les deuxième et troisième raccords (204 ; 203).

6. Dispositif de valve (200) selon l'une quelconque des revendications 1-5, dans lequel le logement (214) comprend un évidement et dans lequel le troisième raccord (203) est disposé dans l'évidement.

7. Dispositif de valve (200) selon l'une quelconque des revendications 1-6, dans lequel le troisième raccord (203) est un raccord à activation Luer.

8. Dispositif de valve (200) selon l'une quelconque des revendications 1-7,
dans lequel les premier et deuxième éléments de valve (206 ; 207) sont encastrés dans le logement (214) et configurés pour empêcher une fermeture accidentelle ou une restriction du trajet de fluide principal.

9. Système visant à drainer des fluides à partir du corps d'un patient et/ou de perfuser des fluides dans celui-ci, comprenant :
un dispositif de valve (200), selon la revendication 1 ;
un élément tubulaire (102, 103) comprenant une première extrémité dimensionnée pour l'introduction dans le corps d'un patient et une deuxième extrémité comprenant un raccord d'élément tubulaire configuré pour s'accoupler au premier raccord (205) ; et
un récipient pour le stockage de fluide à partir du corps du patient comprenant un tube incluant un raccord de récipient configuré pour s'accoupler au deuxième raccord (204), fournissant ainsi un trajet de fluide depuis l'élément tubulaire à travers le trajet de fluide principal et le tube dans le récipient.

10. Système selon la revendication 9, comprenant en outre une source de vide comprenant un raccord de vide configuré pour s'accoupler au troisième raccord (203), à la suite de quoi le trajet de fluide secondaire est ouvert pour fournir un trajet de fluide entre le trajet de fluide principal et la source de vide.

11. Système selon la revendication 10, dans lequel la source de vide comprend une seringue.

12. Système selon la revendication 9, comprenant en outre une source de milieu de perfusion configurée pour s'accoupler au troisième raccord, à la suite de quoi le trajet de fluide secondaire est ouvert pour fournir un trajet de fluide entre le trajet de fluide principal et la source de milieu de perfusion.
